(19)

Europäisches Patentamt

European Patent Office

Office européen des brevets

(11)  **EP 4 682 894 A1**

(12)  **EUROPEAN PATENT APPLICATION**
published in accordance with Art. 153(4) EPC

(43) Date of publication:
**21.01.2026 Bulletin 2026/04**

(21) Application number: **24819223.9**

(22) Date of filing: **28.05.2024**

(51) International Patent Classification (IPC):
**G16C 60/00** (2019.01)    **G16C 20/70** (2019.01)

(52) Cooperative Patent Classification (CPC):
**G16C 20/70; G16C 60/00**

(86) International application number:
**PCT/JP2024/019602**

(87) International publication number:
**WO 2024/252992 (12.12.2024 Gazette 2024/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **05.06.2023  JP 2023092719**

(71) Applicant: **JFE Steel Corporation
Tokyo 100-0011 (JP)**

(72) Inventors:
• **KIYOKANE Naoya
Tokyo 100-0011 (JP)**
• **TAKAJO Shigehiro
Tokyo 100-0011 (JP)**
• **ISHIKAWA Shin
Tokyo 100-0011 (JP)**

(74) Representative: **Scott, Stephen John
YUJA IP LAW
East Coast House
25 Skeldergate
York YO1 6DH (GB)**

(54)  **MANUFACTURING CONDITION SELECTION METHOD, MATERIAL MANUFACTURING METHOD, MANUFACTURING CONDITION SELECTION DEVICE, AND PROGRAM**

(57)    A production condition selection method including: an input process (S11) of inputting an initial data group including initial production condition data and property value data regarding a material to be produced; a search range setting process (S12) of setting a search range for a production condition of the material based on the initial data group; a calculation process (S13) of calculating a predicted property value that is a predicted value of a property value of the material in the search range based on the initial data group, and calculating a prediction error that is an error of the predicted property value, using a machine learning model; and a selection process (S14) of selecting a production condition of the material to be an experimental point based on a calculated value of an acquisition function that uses parameters based on the initial data group, the predicted property value, and the prediction error.

*FIG. 2*

Start

| Input process | S11 |
| Search range setting process | S12 |
| Calculation process | S13 |
| Selection process | S14 |
| Acquisition process | S15 |
| Determination process | S16 |

Pass?  —S17

No / Yes

| Output process | S18 |

End

EP 4 682 894 A1

**Description**

TECHNICAL FIELD

The present disclosure relates to a production condition selection method, a method of producing a material, a production condition selection device, and a program.

BACKGROUND

Properties of metallic materials, for example the mechanical properties of steel materials, are strongly dependent on alloy components added and heat treatment conditions. For example, dual phase (DP) steel sheets are made by adding alloy components such as 0.1 mass% C, 1.0 mass% Si, and 1.0 mass% Mn to a steel sheet, annealing at a temperature in a two-phase range of ferrite and austenite, and then applying a tempering treatment at a temperature of about 200 °C to 400 °C. By such a treatment, both high tensile strength and high ductility (high elongation value) are achievable. Further, the higher the annealing temperature during production of a DP steel sheet, the higher the volume fraction of austenite generally becomes, and as a result, the volume fraction of martensite after quenching becomes higher, and therefore a DP steel sheet having high tensile strength tends to be obtainable. Further, the higher the tempering temperature and the longer the tempering time during production of a DP steel sheet, the more the martensite is tempered and the more the strain in the martensite is relaxed, and therefore a DP steel sheet having low tensile strength and a high elongation value tends to be obtainable. In view of this general trend, steel materials are being developed by adjusting production conditions such as annealing temperature, annealing time, tempering temperature, and tempering time so as to improve mechanical properties such as tensile strength and elongation value. However, these production conditions do not have a clear linear relationship with the mechanical properties, making it difficult to predict optimal production conditions. Further, the optimal production conditions vary depending on type and concentration of alloy components. As a result, the reality is that a huge number of experiments are conducted to comprehensively search for optimal production conditions, and development of steel materials is extremely costly. As a result, technology that uses machine learning to predict material properties is attracting attention.

For example, a method of developing materials using a technique called materials informatics is known. Materials informatics utilizes digital technologies in materials development. Specifically, production conditions for materials to obtain target material properties are predicted from a huge amount of accumulated data on the production conditions and properties of materials.

Further, Patent Literature (PTL) 1 describes a manufacturing evaluation system that automatically carries out sample manufacturing, measurement, and optimization. The manufacturing evaluation system of PTL 1 includes a manufacturing device that manufactures a sample, a measurement device that measures material information representing physical properties or structure of the sample manufactured by the manufacturing device, and an estimation device connected to the manufacturing device and the measurement device. Further, the estimation device includes an estimation unit that estimates an optimal production condition based on a dataset including production conditions and material information of the sample, and a data addition unit that adds to the dataset the production condition estimated by the estimation unit and material information measured from a sample manufactured in accordance with the production condition. Accordingly, the estimation unit can successively estimate the production condition based on the added dataset. As a specific example, the results of an experiment to minimize the electrical resistance of a Nb-doped $TiO_2$ thin film formed on a glass substrate are described. The film was formed by sputtering, and two types of targets, $Ti_{0.94}Nb_{0.06}O_2$ and $Ti_{1.98}Nb_{0.02}O_3$, which have different ratios of Ti and O, were used. By adjusting the mix ratio of Ar gas to a mixed gas of Ar (99 %) and $O_2$ (1 %), the oxygen content (oxygen partial pressure) in the thin film at which the electrical resistance was at a minimum value was sought. At this time, a lower confidence bound was adopted as an acquisition function (A), and the minimum value was searched for as $A = -E + 5\sigma$ (where E is predicted value of material information of the sample and $\sigma$ is predicted error value). Further, upper and lower limits of the oxygen partial pressure, which corresponds to the production condition of the sample, were determined, grid division was executed, and the minimum value of the acquisition function was searched for. In the experiment, the minimum value of the electrical resistance was found after 18 experiments.

CITATION LIST

Patent Literature

PTL 1: JP 2021-101465 A

SUMMARY

(Technical Problem)

Typically, material development methods using materials informatics are based on the premise that a huge amount of data on the production conditions and properties of materials already exists, and therefore there is a problem that the number of experimental steps becomes large and efficiency is low.

In the method described in PTL 1, a linear addition of a predicted value and a prediction error is used as the acquisition function (A), and the probability (E) of achieving the predicted value is treated as equivalent to the probability of achieving a property that is $5\sigma$ away from the predicted value. This poses a problem of low efficiency, as the number of experimental steps required to find optimal sample production conditions becomes large.

In view of the above circumstances, it would be helpful to provide a production condition selection method, a method of producing a material, a production condition selection device, and a program that can efficiently select a production condition for improving a property value of a material.

(Solution to Problem)

[1] A production condition selection method according to an embodiment of the present disclosure comprises:

an input process of inputting an initial data group including initial production condition data and property value data regarding a material to be produced;

a search range setting process of setting a search range for a production condition of the material based on the initial data group;

a calculation process of calculating a predicted property value that is a predicted value of a property value of the material in the search range based on the initial data group, and calculating a prediction error that is an estimated error of the predicted property value, using a machine learning model; and

a selection process of selecting a production condition of the material to be an experimental point based on a calculated value of an acquisition function that uses parameters based on the initial data group, the predicted property value, and the prediction error.

[2] As an embodiment of the present disclosure, [1], further comprising:

an acquisition process of acquiring a property value of the material under the production condition as the experimental point; and

a determination process of determining, by comparing calculated values of the acquisition function before and after adding the property value of the material acquired and the production condition as the experimental point to the initial data group, whether or not to select a further production condition of the material to be a new experimental point.

[3] As an embodiment of the present disclosure, [1] or [2], wherein the acquisition function is expressed by the following Expression (1), where A is the calculated value, R is a random number corresponding to a magnitude of a measurement error for correcting the measurement error when measuring the property value of the material, $y_{av}$ is the predicted property value, $y_{\sigma}$ is an error of the predicted property value, Y is a measured value of the property value, $Y_{\sigma}$ is a standard deviation of the measured value, $\max(Y)$ is the maximum value of the measured value, and $\varepsilon_1$, $\varepsilon_2$, and $\varepsilon_3$ are parameters for correcting the predicted error.

[Math. 1]

$$A = (y_{av} - R \cdot max(Y) - \varepsilon_1 \cdot Y_{\sigma}) \cdot \int_{R \cdot max(Y)}^{+\infty} \frac{1}{\sqrt{(\varepsilon_2 \cdot y_{\sigma})}} exp\left\{-\frac{1}{\varepsilon_3 \cdot y_{\sigma}}(y - y_{av} - \varepsilon_1 \cdot Y_{\sigma})^2\right\} dy + \varepsilon_2 \cdot y_{\sigma} \cdot \frac{1}{\sqrt{(\varepsilon_2 \cdot y_{\sigma})}} exp\left\{-\frac{1}{\varepsilon_3 \cdot y_{\sigma}}(max(Y) - y_{av} - \varepsilon_1 \cdot Y_{\sigma})^2\right\}$$

...Expression (1)

[4] A method of producing a material according to an embodiment of the present disclosure comprises:
producing the material using a production condition selected by the production condition selection method according to any one of [1] to [3].

[5] A production condition selection device according to an embodiment of the present disclosure comprises:

an input interface configured to input an initial data group including initial production condition data and property value data regarding a material to be produced;

a search range setting unit configured to set a search range for a production condition of the material based on the initial data group;

a calculation unit configured to calculate a predicted property value that is a predicted value of a property value of the material in the search range based on the initial data group, and calculate a prediction error that is an estimated error of the predicted property value, using a machine learning model; and

a selection unit configured to select a production condition of the material to be an experimental point based on a calculated value of an acquisition function that uses parameters based on the initial data group, the predicted property value, and the prediction error.

[6] A program according to an embodiment of the present disclosure is configured to
cause a computer to function as:

an input interface configured to input an initial data group including initial production condition data and property value data regarding a material to be produced;

a search range setting unit configured to set a search range for a production condition of the material based on the initial data group;

a calculation unit configured to calculate a predicted property value that is a predicted value of a property value of the material in the search range based on the initial data group, and calculate a prediction error that is an estimated error of the predicted property value, using a machine learning model; and

a selection unit configured to select a production condition of the material to be an experimental point based on a calculated value of an acquisition function that uses parameters based on the initial data group, the predicted property value, and the prediction error.

(Advantageous Effect)

According to the present disclosure, it is possible to provide a production condition selection method, a method of producing a material, a production condition selection device, and a program that can efficiently select a production condition for improving a property value of a material.

BRIEF DESCRIPTION OF THE DRAWINGS

In the accompanying drawings:

FIG. 1 is a schematic diagram illustrating a configuration example of a production condition selection system including a production condition selection device according to an embodiment of the present disclosure;

FIG. 2 is a flowchart illustrating processing of a production condition selection method according to an embodiment of the present disclosure;

FIG. 3 is a diagram illustrating a distribution of property values based on measured values obtained by experiment for Example Group 1;

FIG. 4 is a diagram illustrating a relationship between number of steps and maximum value of property values for Example Group 1;

FIG. 5 is a diagram illustrating distributions of property values according to number of steps for Example 1;

FIG. 6 is a diagram illustrating distributions of property values according to number of steps for Example 2;

FIG. 7 is a diagram illustrating distributions of property values according to number of steps for Comparative Example 1;

FIG. 8A is a diagram illustrating distribution of a plurality of property values based on measured values obtained by experiment for Example Group 2;

FIG. 8B is a diagram illustrating distribution of a plurality of property values based on measured values obtained by experiment for Example Group 2;

FIG. 8C is a diagram illustrating distribution of a plurality of property values based on measured values obtained by experiment for Example Group 2;

FIG. 9 is a diagram illustrating a relationship between number of steps and maximum value of property values for Example Group 2;

FIG. 10 is a diagram illustrating distribution of property values according to number of steps for Example 3;

FIG. 11 is a diagram illustrating distributions of property values according to number of steps for Example 4;

FIG. 12 is a diagram illustrating distributions of property values according to number of steps for Comparative Example 2; and

FIG. 13 is a diagram illustrating distributions of property values according to number of steps for Comparative Example 3.

DETAILED DESCRIPTION

Hereinafter, a production condition selection method, a method of producing a material, a production condition selection device, and a program according to embodiments of the present disclosure are described, with reference to the drawings. In each drawing, identical or equivalent parts are marked with the same reference sign. In description of the following embodiments, description of identical or equivalent parts is omitted or simplified as appropriate.

(Production condition selection system)

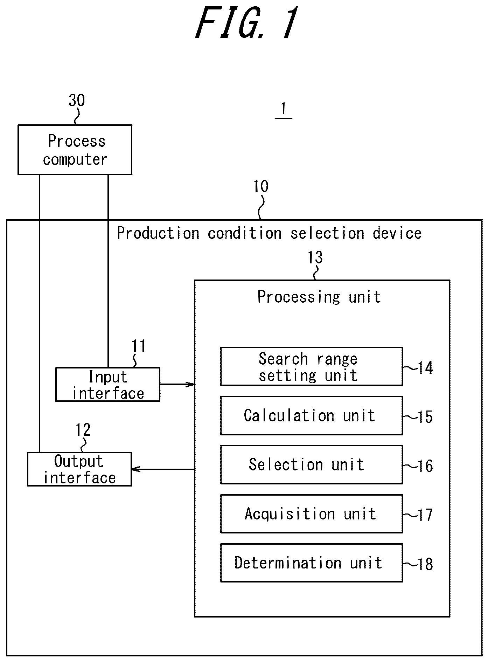

FIG. 1 is a block diagram of a production condition selection system 1 including a production condition selection device 10 according to the present embodiment. The production condition selection system 1 includes the production condition selection device 10. The production condition selection device 10 includes an input interface 11, an output interface 12, and a processing unit 13. The processing unit 13 includes a search range setting unit 14, a calculation unit 15, a selection unit 16, an acquisition unit 17, and a determination unit 18. The production condition selection system 1 may further include a process computer 30 as in the configuration example of FIG. 1. Here, the configuration of the production condition selection system 1 is not limited to that illustrated in FIG. 1. For example, the production condition selection system 1 may further include a display (display device) that displays data from the output interface 12. Further, the production condition selection system 1 may include a server computer that aggregates data in addition to or instead of the process computer 30. In such a case, an operator may be able to input data to the server computer via an input device (such as a mouse or keyboard), and the input data may be transferred from the server computer to the input interface 11. Further, the process computer 30 may be a computer positioned above a plurality of control computers, each of which controls a specific production device. In such a case, data may be input to the input interface 11 directly from some or all of the control computers.

(Process computer)

The process computer 30 is a computer that controls production apparatus in a line (production line) that produces a production object, or a computer that controls experimental conditions related to production in a laboratory. The production object may be, for example, a metal material such as a steel material, but is not limited to a metal material and may be a general material. The process computer 30 may also function as a data server that aggregates data related to production. Here, as described above, a server computer that aggregates data related to production may be used in place of the process computer 30. Data related to production includes, for example, production conditions and measured values obtained by measuring produced material. The process computer 30 may be capable of communicating with the production condition selection device 10 via a network.

(Production condition selection device)

The production condition selection device 10 is a device that selects a production condition so as to improve a property value of the material to be produced. The property value is a value that represents a property of a material, and may be, for example, in the case of a metal material, strength, proof stress, elongation, hardness, a value obtained by combining these, or the like.

(Input interface)

The input interface 11 is an input interface of the production condition selection device 10. An initial data group including initial production condition data and property value data of the material to be produced is input to the input interface 11. Here, "initial" indicates already set, already measured, or already predicted. That is, the initial data group includes an initial production condition, which is a production condition, and a property value that has already been obtained by actual measurement or prediction (calculation) regarding the material. The input interface 11 executes an input process that is described later. Further, production conditions include not only conditions on a production line but also experimental conditions in a laboratory.

(Output interface)

The output interface 12 is an output interface of the production condition selection device 10. The output interface 12 may output a production condition selected by the production condition selection device 10 to the process computer 30. Further, the output interface 12 may cause a production condition selected by the production condition selection device 10 to be displayed on a display device such as any display so that an operator of a production line can confirm the production condition. The output interface 12 executes an output process, which is described later.

(Processing unit)

The processing unit 13 executes calculations for the production condition selection device 10 to select a production condition. Further, the processing unit 13 may function as a control unit that controls the production condition selection device 10 as a whole. The processing unit 13 may be one or more processors. Each processor may be, but is not limited to, a general-purpose processor or a dedicated processor specialized for particular processing, and may be any processor.

As described above, according to the present embodiment, the processing unit 13 includes the search range setting unit 14, the calculation unit 15, the selection unit 16, the acquisition unit 17, and the determination unit 18. Functions of the search range setting unit 14, the calculation unit 15, the selection unit 16, the acquisition unit 17, and the determination unit 18 may be realized by software. For example, one or more programs may be stored in a storage device accessible by the processing unit 13. When a program stored in the storage device is read by the processing unit 13, which is a processor, the program may cause the processing unit 13 to function as the search range setting unit 14, the calculation unit 15, the selection unit 16, the acquisition unit 17, and the determination unit 18.

(Search range setting unit)

The search range setting unit 14 sets a search range for a production condition of the material based on the initial data group. That is, the search range setting unit 14 limits a range of a production condition to be selected. Details of processing executed by the search range setting unit 14 are described later. Further, the search range setting unit 14 executes a search range setting process that is described later.

(Calculation unit)

The calculation unit 15 uses a model to calculate a predicted property value that is a predicted value of a property value of the material in a search range based on an initial data group, and a prediction error that is an estimated error of the predicted property value. According to the present embodiment, the model is a machine learning model. Details of processing executed by the calculation unit 15 are described later. Further, the calculation unit 15 executes a calculation process that is described later.

(Selection unit)

The selection unit 16 selects a production condition of the material to be an experimental point based on a calculated value of an acquisition function that uses parameters based on the initial data group, the predicted property value, and the prediction error. Experimental points are specific production conditions under which an experiment (according to the present embodiment, production of a material) was or will be carried out. The experimental point selected by the selection unit 16 is preferably a production condition that is not included in the initial production condition data, that is, a production condition that has not been tested so far. Details of processing executed by the selection unit 16 are described later. Further, the selection unit 16 executes a selection process that is described later.

(Acquisition unit)

The acquisition unit 17 acquires a property value of the material under a production condition as an experimental point. Details of processing executed by the acquisition unit 17 are described later. Further, the acquisition unit 17 executes an acquisition process that is described later.

(Determination unit)

The determination unit 18 determines, by comparing calculated values of an acquisition function before and after adding a property value of the material acquired and a production condition as an experimental point to an initial data group, whether or not to select a further production condition of the material to be a new experimental point. Here, no need to select a production condition of the material as a new experimental point is sometimes referred to as a "pass". That is, the determination unit 18 determines a "pass", which means that selection of new experimental point is not necessary, or a "fail", which means that selection of a new experimental point is necessary. Details of processing executed by the determination unit 18 are described later. Further, the determination unit 18 executes a determination process that is described later.

Here, the production condition selection device 10 is not limited to a specific device, and as an example may be realized by a computer. The computer may be a commercially available, general-purpose computer, for example. The computer may, for example, include a storage device, such as memory and a hard disk drive, a CPU, and input/output devices. The processing unit 13 may be realized by a CPU. A program to be read by the processing unit 13 may be stored in a storage device. The input interface 11 and the output interface 12 may be realized by input/output devices.

(Production condition selection method)

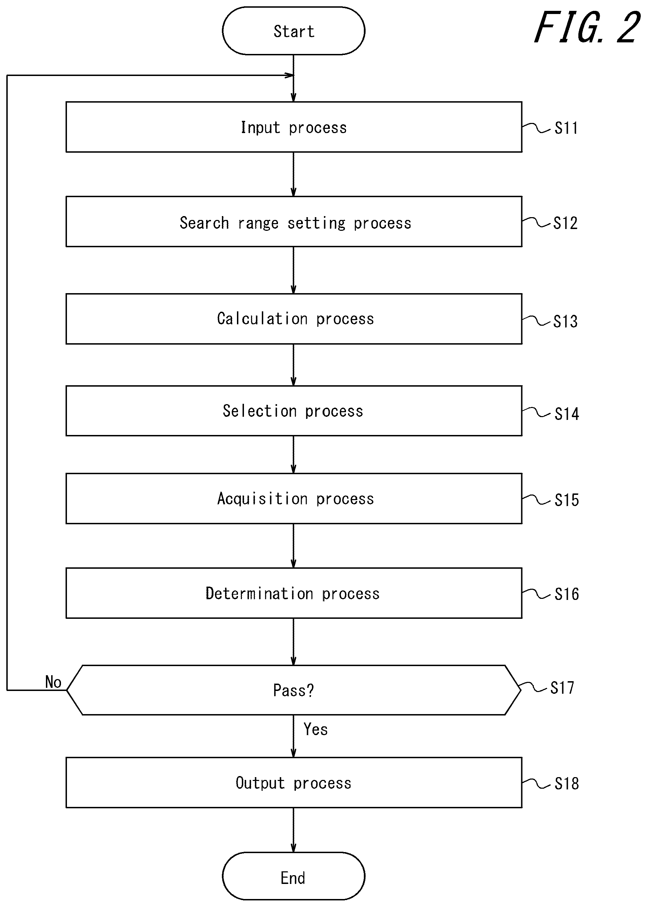

FIG. 2 is a flowchart illustrating processing of the production condition selection method executed by the production condition selection device 10 according to the present embodiment. The production condition selection method includes the input process, the search range setting process, the calculation process, and the selection process. The production condition selection method may further include the acquisition process and the determination process.

(Input process)

In the input process, an initial data group including initial production condition data and property value data of the material is input (step S11).

As described above, the initial data group is a data group that includes existing initial production condition data and property value data of the material obtained under the production condition.

The production condition data is a control variable that is controlled in order to produce the material to be produced. For example, when the material to be produced is a DP steel sheet, the production condition data is chemical composition, annealing temperature, annealing time, tempering temperature, or the like.

The property value data of the material is a property value of the material to be improved. For example, when the material to be produced is a DP steel sheet, the property value data of the material is yield stress, tensile strength, elongation value, or the like.

The initial data group may be one or more sets. A set is a combination of a production condition and a property value of the material produced under the production condition. In order to efficiently predict a production condition that will result in a better property value, the initial data group is preferably two or more sets. The initial data group is more preferably four or more sets. It is preferable to have as many sets of initial data as possible.

When the number of sets in the initial data group is small, in order to efficiently predict (that is, with fewer experimental steps) a production condition that will result in a better property value, it is preferable that the initial data group include sets that correspond to ends (near boundaries) of the search range described below.

(Search range setting process)

In the search range setting process, a search range for a production condition of the material is set based on the initial data group (step S12).

Specifically, an upper limit and a lower limit of a search range for searching a production condition of the material to be an experimental point are set such that a property value of the material may be improved over the property value of the initial data group. By setting the search range, a production condition (experimental point) of the material to efficiently achieve an excellent property value may be predicted.

A calculated value of an acquisition function used in the selection process described later has a property of becoming larger the further away it is from the production conditions of data points input as the initial data group. When a search range is not set, this property of the acquisition function may result in the selection of an experimental point that is greatly different from the production conditions of the data points entered as the initial data group, which may result in divergence of calculation. Therefore, a search range is set. The upper limit and the lower limit of the search range may be set based on initial production condition data, and may also be set in consideration of conventional knowledge (for example, widely known theories or knowledge in the production of the material).

For example, in development of a steel material, a search range may be set so that an annealing temperature that is so low that carbon diffusion cannot occur and, as a result, material microstructure does not change at all, is not selected. Further, for example, in development of a DP steel sheet having an excellent balance between strength and ductility, the upper limit and the lower limit of the search range for a production condition such as chemical composition, annealing temperature, or the like is set. For example, based on conventional knowledge such as literature on DP steel sheets, the $A_1$ point may be selected as the lower limit of the annealing temperature, and the $A_4$ point may be selected as the upper limit of the annealing temperature. It is known that when the annealing temperature is below the $A_1$ point, the phase does not transform into austenite, and therefore the microstructure does not change at all, and an improvement in the balance between strength and ductility by annealing cannot be expected. Further, when the annealing temperature exceeds the $A_4$ point, $\delta$-ferrite precipitates. Therefore, it is known that when the annealing temperature exceeds the $A_4$ point, then even when the microstructure is subsequently converted to full austenite by cooling, the microstructure will become full martensite by quenching, and therefore an improvement in the balance between strength and ductility cannot be expected. In this way, by using conventional knowledge of a material to eliminate conditions that do not indicate an improved property, it is possible to predict a production condition for the material that will efficiently achieve an excellent property value. Therefore, it is preferable to select an experimental condition within a reasonable range based on material knowledge. However, when searching a region that differs from conventional knowledge, that is, differs from general assumptions in the field of materials, the search range may be set without being bound by conventional knowledge.

Here, the search range may be a continuous space, but in order to achieve an improvement in a property value of the material with a small number of experimental steps, it is preferable that the search range be a discrete space. When the search range is a discrete space, the number of production conditions (search points) of the material to be searched for can be reduced compared to when the search range is a continuous space.

(Calculation process)

In the calculation process, a predicted property value and a predicted error of the material in the search range are calculated using a model based on the initial data group (step S13).

Specifically, using the initial data group input in the input process, the production condition of the material, which is the control variable, is changed within the search range set in the search range setting process to calculate the predicted property value and the prediction error of the material. For example, in the case of a DP steel sheet, a predicted material property value such as yield stress, tensile strength, or the like is calculated for a control variable such as chemical composition, annealing temperature, annealing time, or the like.

A model, such as a machine learning model, may be used to calculate the predicted property value of the material. The machine learning model may be generated using a neural network model, a Gaussian process model, random forests, or the like. For example, a plurality of sets of production conditions and property values of materials produced under the production condition may be extracted from actual performance data related to production aggregated in the process computer 30 and used as training data. The model used to calculate the predicted property value of the material may be an interpolation function generated based on an initial data group, such as a known linear approximation, spline interpolation, or the like. When a plurality of models are used in the calculation process, an average value of the predicted property values calculated using the plurality of models may be adopted as a final predicted property value.

Further, the error (prediction error) of the calculated predicted property value of the material is calculated. For example, the prediction error may be the standard deviation of predicted property values calculated using a plurality of models. Further, for example, the prediction error may be the standard deviation of predicted property values calculated by changing the combination of the initial data group input using one model. Further, when a prediction error can be obtained from one model, such as calculation using a Gaussian process model, the obtained prediction error may be used as is.

(Selection process)

In the selection process, a production condition of the material to be an experimental point is selected based on a calculated value of an acquisition function obtained using the initial data group, the predicted property value of the material, and the prediction error (step S14).

The selected experimental point is, for example, a production condition that is an optimal solution where the acquisition function indicates a maximum value or a minimum value (maximum value according to the present embodiment). However, the selected experimental point may be a production condition of a suboptimal solution (for example, the top 10 % of the acquisition function). For example, for a problem with a small measurement error, selecting an experimental point where experiments have already been conducted is typically meaningless and inefficient. Therefore, when the optimal solution where the acquisition function indicates a maximum value or a minimum value coincides with an experimental point where an experiment has already been conducted, a suboptimal solution may be selected as the experimental point. However, for a problem with a large measurement error, when the optimal solution coincides with an experimental point where an experiment has already been conducted, the optimal solution may be selected as the experimental point in order to check reproducibility.

Typically, the acquisition function used is upper confidence bound (UCB) or probability of improvement (PI). In the production condition selection method according to the present embodiment, the acquisition function indicated in Expression (1) is used to improve the efficiency of selection. A is the calculated value of the acquisition function.

[Math. 2]

$$A = (y_{av} - R \cdot max(Y) - \varepsilon_1 \cdot Y_\sigma) \cdot \int_{R \cdot max(Y)}^{+\infty} \frac{1}{\sqrt{(\varepsilon_2 \cdot y_\sigma)}} exp\left\{-\frac{1}{\varepsilon_3 \cdot y_\sigma}(y - y_{av} - \varepsilon_1 \cdot Y_\sigma)^2\right\} dy + \varepsilon_2 \cdot y_\sigma \cdot \frac{1}{\sqrt{(\varepsilon_2 \cdot y_\sigma)}} exp\left\{-\frac{1}{\varepsilon_3 \cdot y_\sigma}(max(Y) - y_{av} - \varepsilon_1 \cdot Y_\sigma)^2\right\}$$

...Expression (1)

Here, R is a random number. More specifically, R is a parameter for correcting a measurement error when measuring a property value of a material, and is a random number according to the magnitude of the measurement error. $y_{av}$ is the predicted value (predicted property value). $y_\sigma$ is the error of the predicted value (predicted property value). Y is the measured value of the property value. $Y_\sigma$ is the standard deviation of the measured value. max(Y) is the maximum value of the measured value. $\varepsilon_1$, $\varepsilon_2$, and $\varepsilon_3$ are parameters. In detail, $\varepsilon_1$, $\varepsilon_2$, and $\varepsilon_3$ are parameters that correct the prediction error.

Here, for example, in the case of a steel material, there may typically be a measurement error of about 1 % for total elongation. Therefore, when the total elongation is used as the property value, the measurement error can be corrected by generating the random number R in the range from 0.99 to 1.01. When the measurement error is small, R may be set to 1.0.

Further, $\varepsilon_1$, $\varepsilon_2$, and $\varepsilon_3$ are preferably 0.01 or more. $\varepsilon_1$, $\varepsilon_2$, and $\varepsilon_3$ are more preferably 0.1 or more. $\varepsilon_1$, $\varepsilon_2$, and $\varepsilon_3$ are even more preferably 0.5 or more. When $\varepsilon_1$, $\varepsilon_2$, and $\varepsilon_3$ are less than 0.1, the influence of the prediction error becomes excessively small, and the number of experimental steps required until a pass determination increases, as described later. Further, $\varepsilon_1$, $\varepsilon_2$, and $\varepsilon_3$ are preferably 10.0 or less. $\varepsilon_1$, $\varepsilon_2$, and $\varepsilon_3$ are more preferably 5.0 or less. When $\varepsilon_1$, $\varepsilon_2$, and $\varepsilon_3$ are made larger than 10.0, the influence of the prediction error becomes excessively large, and the number of experimental steps required until a pass determination increases, as described later.

When it is necessary to improve two or more property values, it is preferable to use the acquisition functions indicated in Expressions (3) and (4) in the production condition selection method according to the present embodiment.

[Math. 3]

$$A = \prod_{i=1}^{N}\left\{\frac{A_i}{max(A_i)}\right\}$$

...Expression (3)

$$A_i = (y_{av,i} - R_i \cdot max(Y_i) - \varepsilon_{1,i} \cdot Y_{\sigma,i}) \times \int_{R_i \cdot max(Y_i)}^{+\infty} \frac{1}{\sqrt{(\varepsilon_{2,i} \cdot y_{\sigma,i})}} exp\left\{-\frac{1}{\varepsilon_{3,i} \cdot y_{\sigma,i}}(y_i - y_{av,i} - \varepsilon_{1,i} \cdot Y_{\sigma,i})^2\right\} dy_i + \varepsilon_2 \cdot y_{\sigma,i} \cdot \frac{1}{\sqrt{(\varepsilon_{2,i} \cdot y_{\sigma,i})}} exp\left\{-\frac{1}{\varepsilon_{3,i} \cdot y_{\sigma,i}}(max(Y_i) - y_{av,i} - \varepsilon_{1,i} \cdot Y_{\sigma,i})^2\right\}$$

...Expression (4)

Here, i is each parameter corresponding to property value i. The magnitude of the absolute value of the acquisition function $A_i$ corresponding to each property value changes depending on the magnitude of the property value. Therefore, when an acquisition function is calculated by multiplying the acquisition function corresponding to the respective property value, the influence of a property value with a large absolute value is large, and there is a risk that a property value with a small absolute value is not taken into account. Therefore, it is preferable to calculate the acquisition function A after normalizing by the maximum value $max(A_i)$ of the acquisition function $A_i$ corresponding to each property value.

Here, $R_i$ is a random number corresponding to the property value i. More specifically, $R_i$ is a parameter for correcting a measurement error when measuring a property value i of the material, and is a random number according to the magnitude of the measurement error. $y_{av,i}$ is the predicted value (predicted property value) of property value i. $y_{\sigma,i}$ is the error of the predicted value (predicted property value) of property value i. $Y_i$ is the measured value of property value i. $Y_{\sigma,i}$ is the standard deviation of the measured values of property value i. $max(Y_i)$ is the maximum value of the measured values of property value i. Each of $\varepsilon_{1,i}$, $\varepsilon_{2,i}$, and $\varepsilon_{3,i}$ is a parameter corresponding to property value i. In detail, $\varepsilon_{1,i}$, $\varepsilon_{2,i}$, and $\varepsilon_{3,i}$ are parameters that correct the prediction error.

Here, for example, in the case of a steel material, there may typically be a measurement error of about 1 % for total elongation. Therefore, when the total elongation is used as the property value, the measurement error can be corrected by generating the random number $R_i$ in the range from 0.99 to 1.01. When the measurement error is small, $R_i$ may be set to 1.0.

Further, $\varepsilon_{1,i}$, $\varepsilon_{2,i}$, and $\varepsilon_{3,i}$ are preferably 0.01 or more. $\varepsilon_{1,i}$, $\varepsilon_{2,i}$, and $\varepsilon_{3,i}$ are more preferably 0.1 or more. $\varepsilon_{1,i}$, $\varepsilon_{2,i}$, and $\varepsilon_{3,i}$ are even more preferably 0.5 or more. When $\varepsilon_{1,i}$, $\varepsilon_{2,i}$, and $\varepsilon_{3,i}$ are less than 0.1, the influence of the prediction error becomes excessively small, and the number of experimental steps required until a pass determination increases, as described later. Further, $\varepsilon_{1,i}$, $\varepsilon_{2,i}$, and $\varepsilon_{3,i}$ are preferably 10.0 or less. $\varepsilon_{1,i}$, $\varepsilon_{2,i}$, and $\varepsilon_{3,i}$ are more preferably 5.0 or less. When $\varepsilon_{1,i}$, $\varepsilon_{2,i}$, and $\varepsilon_{3,i}$ are made larger than 10.0, the influence of the prediction error becomes excessively large, and the number of experimental steps required until a pass determination increases, as described later.

(Acquisition process)

In the acquisition process, the property value of the material under the production condition of the material as the experiment point selected in the selection process is acquired (step S15).

In the acquisition process, the data points obtained in the selection process, that is, the selected experimental point and the property value of the material corresponding to the experimental point (set of data), are added to the initial data group. To improve the prediction precision of the property value, a machine learning model may be generated (updated) using the initial data group to which a set of data has been added.

In the acquisition process, it is preferable that a material is actually produced at the selected experimental point (production condition) and the property value of the produced material is actually measured. In other words, it is preferable that the property value added to the initial data group is a measured value. However, the property value added to the initial data group may be a predicted property value calculated using a machine learning model or the like.

(Determination process)

In the determination process, based on the calculated values of the acquisition function obtained before and after adding the selected experimental point (production condition) and the property value acquired in the acquisition process to the initial data group, it is determined whether or not to select a production condition of the material to be a new experimental point (step S16).

The calculated value of the acquisition function obtained using Expression (1) from the initial data group input in the input process is ($A_1$). Further, the calculated value of the acquisition function obtained by using Expression (1) from the initial data group after adding the data points representing the property value and the production condition obtained in the acquisition process is ($A_2$). In the determination process, ($A_1$) and ($A_2$) are compared to determine whether or not to search for a production condition as a next experimental point.

For example, for the production condition in the search range, the difference between ($A_1$) and ($A_2$) is calculated, and the difference is divided by the number of data points (the number of search points) to obtain a normalized difference. When the normalized difference is less than a threshold value, a sufficient improvement in the property value has been obtained, which is determined as a pass (Yes in step S17), and a search for a new experimental point is not executed. The threshold value may be 0.001 as an example, but is not limited to a specific value and may be set according to the type of material, for example. When the normalized difference is equal to or greater than the threshold value, this is determined as a fail (No in step S17), and a search for a new experimental point is executed. That is, in the case of a fail, processing returns to the input process (step S11) and the series of processing is executed again. Here, in the case of a fail, the initial data group input in the input process becomes the initial data group after the set of data is added in the acquisition process.

(Output process)

When the determination process determines a pass (Yes in step S17), the output process is executed (step S18). The output process may output the pass/fail of the determination process, as well as the property value and the production condition thereof obtained in the acquisition process.

Here, the production condition selection method according to the present embodiment may be realized by installing software for executing the processing of the production condition selection method on a computer. Further, the production condition selection method according to the present embodiment may be implemented by installing software on a cloud computer on a network.

In this way, the production condition selection method executed by the production condition selection device 10 according to the present embodiment makes it possible to efficiently select a production condition for improving a property value of the material. The production condition selection method may be executed as part of a method of producing a material to be produced. For example, the process computer 30 may acquire a production condition selected by the production condition selection method executed by the production condition selection device 10, and execute a control so as to produce a material using the acquired production condition.

EXAMPLES

Advantageous effects of the present disclosure are described in detail below based on examples, although the present disclosure is not limited to these examples. In the examples, the production condition selection method was carried out using the production condition selection device 10 described above.

(Example Group 1)

In Example Group 1, the material to be produced was mild steel that is required to be soft and ductile. In Example Group 1, the production condition was selected to obtain mild steel that has one property, that is, more soft and ductile. The property of being soft and ductile is evaluated to be better when a ratio (El/YS) is larger, which is the ratio of total elongation value (El) to yield stress (YS), which are property values of the material. In the production conditions of the mild steel, the tempering temperature and the tempering time were changed. There were four combinations of tempering temperature and tempering time (first to fourth conditions) as follows, and test materials 1 to 4 were produced under each set of the conditions.

Under the first condition, (normalized tempering temperature, normalized tempering time) were set to (-0.3, -0.3). Under the second condition, (normalized tempering temperature, normalized tempering time) were set to (-0.3, -0.1). Under the third condition, (normalized tempering temperature, normalized tempering time) were set to (0.3, 0.1). Under the fourth condition, (normalized tempering temperature, normalized tempering time) were set to (0.3, 0.3). In order to efficiently find the maximum value of the property, these initial points were set to include the edges of the search space, which is described later.

Here, the normalized tempering temperature was tempering temperature, normalized. The normalization was performed by determining the average value and standard deviation of the tempering temperatures of 30 experimental points in the mild steel database obtained so far, subtracting the obtained average value from the tempering temperature, and dividing by the standard deviation. Further, the normalized tempering time was tempering time, normalized. The normalization was performed by determining the average value and standard deviation of the tempering times of 30 experimental points in the mild steel database obtained so far, subtracting the obtained average value from the tempering time, and dividing by the standard deviation.

For the test materials 1 to 4, JIS No. 5 test pieces 1 to 4 were produced and subjected to tensile tests to measure the yield stress (YS) and total elongation value (El) of the test materials 1 to 4. The obtained production conditions of the material and the property values (El/YS) of the material produced under those production conditions were used as an initial data group (corresponding to step S11). The property value (El/YS) was also normalized. In normalizing (El/YS), first an average value of experimental points of El over 30 points in a database of mild steel was determined, and then El was divided by the average value to calculate the normalized El (norm.El). Similarly, the normalized YS (norm.YS) was calculated by dividing the average value of experimental points of YS over 30 points. Then, norm.El/norm.YS was calculated to normalize (El/YS). Hereinafter, all references to property value (El/YS) refer to this normalized norm.El/-norm.YS.

Next, a search range was set (corresponding to step S12). In this Example Group, the ends of the normalized tempering temperature and normalized tempering time of the initial data group were set as the upper and lower limits of the search ranges of the normalized tempering temperature and normalized tempering time. That is, the upper limit of the search range for the normalized tempering temperature was set to 0.3, and the lower limit was set to -0.3. Further, the upper limit of the search range for the normalized tempering time was set to 0.3, and the lower limit was set to -0.3.

In order to suppress an increase in the number of experimental steps, the search range was set to be a discrete space divided in increments of 0.01 for the normalized tempering temperature and the normalized tempering time.

Next, the predicted property value and the prediction error were calculated (corresponding to step S13). The predicted property value and the prediction error were calculated using a Gaussian process model using the initial data group.

Next, a calculated value ($A_1$) of the acquisition function of Expression (1) was determined using the initial data group and the obtained predicted property value and prediction error. The production condition under which the calculated value ($A_1$) of the acquisition function was maximized was selected as the experimental point (corresponding to step S14). Hereinafter, the selected production condition may be referred to as "production condition (1)".

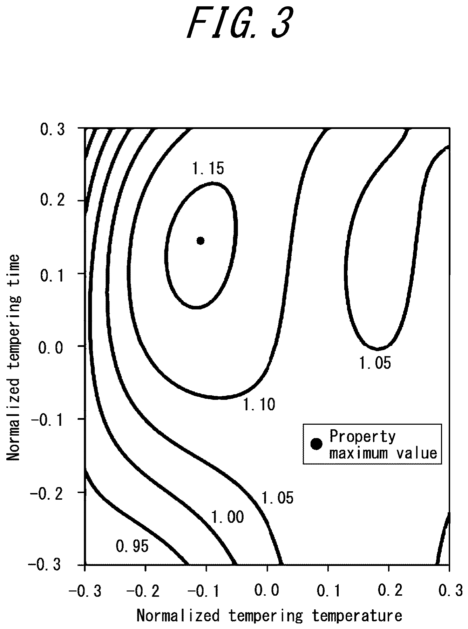

Next, the property value (El/YS) under the production condition of the selected experimental point was acquired (corresponding to step S15). Hereinafter, the acquired property value (El/YS) may be referred to as "property value (1)". The property value (1) here was acquired from FIG. 3 as illustrated below.

FIG. 3 is a diagram illustrating the distribution of the property value based on measured values. The distribution in FIG. 3 was calculated by creating a spline interpolation function based on the property value (El/YS) at 30 points measured experimentally. In FIG. 3, the maximum property value, which is the maximum value of the property value (El/YS), was calculated to be "1.16". Further, in FIG. 3 and FIG. 5 to FIG. 7 described later, the distributions of the property values are illustrated in graphs with the horizontal axis representing the normalized tempering temperature and the vertical axis representing the normalized tempering time. In FIG. 3, the coordinates (normalized tempering temperature, normalized tempering time) corresponding to the property maximum value are (-0.11, 0.14). Here, since there was no measurement error, the R of the acquisition function defined by Expression (1) was set to 1.0.

Next, based on the calculated value ($A_2$) of the acquisition function obtained after adding the production condition (1) and the property value (1) of the obtained material to the initial data group, and the calculated value ($A_1$) of the acquisition function described above, it was determined whether or not to search for a production condition to be the next experimental point (corresponding to step S16). Specifically, for the production condition in the search range, the difference between ($A_1$) and ($A_2$) was calculated, and the difference was divided by the data points to calculate a normalized difference. Thereafter, when the normalized difference was 0.001 or more (equal to or greater than the threshold value), it was determined to be a fail (corresponding to No in step S17), the property value (1) and the production condition (1) were added to the initial data group, and the processing corresponding to the above steps S11 to S16 was executed. Thereafter, the processing corresponding to steps S11 to S16 described above was repeatedly executed until the normalized difference became less than 0.001 (less than the threshold value).

For Example 1, for the acquisition function of Expression (1) used in the selection process, $\varepsilon_1$ was set to 1.0, and $\varepsilon_2$ and $\varepsilon_3$ were set to 2.0. For Example 2, for the acquisition function of Expression (1) used in the selection process, $\varepsilon_1$ was set to 1.0, and $\varepsilon_2$ and $\varepsilon_3$ were set to 0.1.

For Comparative Example 1, UCB (Expression (2)) was used as the acquisition function, and the coefficient term ($\varepsilon_U$) of the prediction error value was set to 6.0. Here, $y_{av}$ was the predicted value (predicted property value), and $y_\sigma$ was the error of the predicted value (predicted property value).

[Math. 4]

$$UCB = -y_{av} + \varepsilon_U \cdot y_\sigma \qquad \ldots \text{Expression (2)}$$

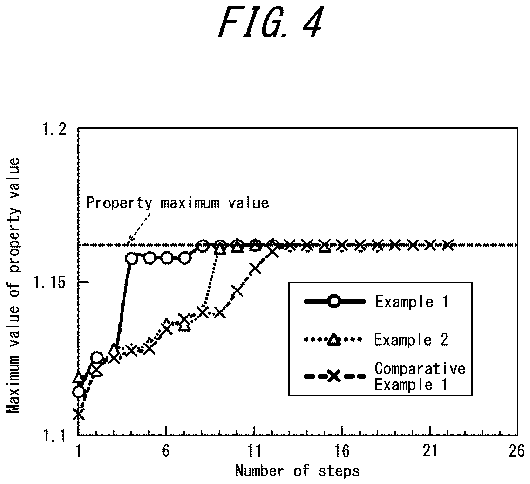

FIG. 4 illustrates the relationship between the number of steps and the maximum value of the property value when produced under the production condition proposed by this model. The property values here were acquired by interpolating with the spline function illustrated in FIG. 3. The number of steps is the number of times the processing from the input process to the determination process was executed, and may also be called the number of trials. In FIG. 4, for example, when the number of steps is 6, the maximum value of the property values obtained in steps 1 to 6 is indicated. The number of steps required to reach the property maximum value was 12 for Comparative Example 1, whereas for Example 1, the property maximum value was approached in 4 steps and reached in 8 steps. For Example 2, the property maximum value was reached in 9 steps, and it can be seen that the number of steps was fewer for the Examples than the Comparative Example.

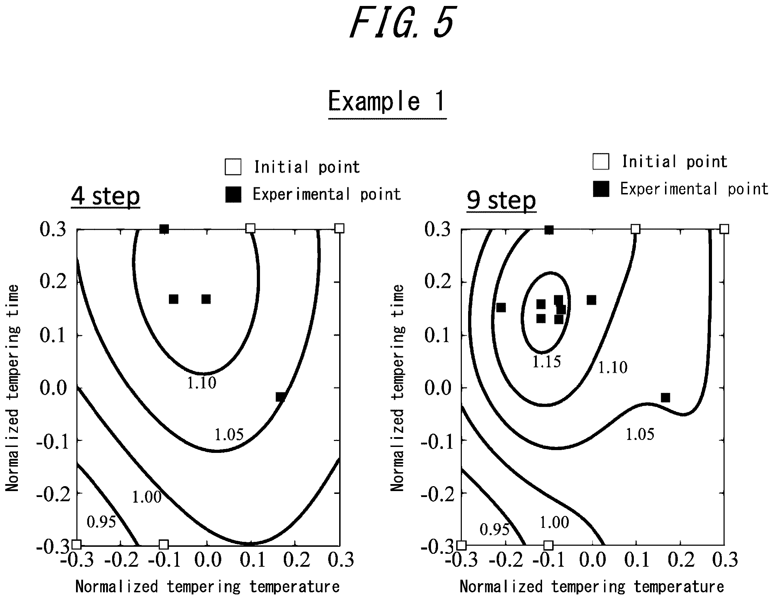

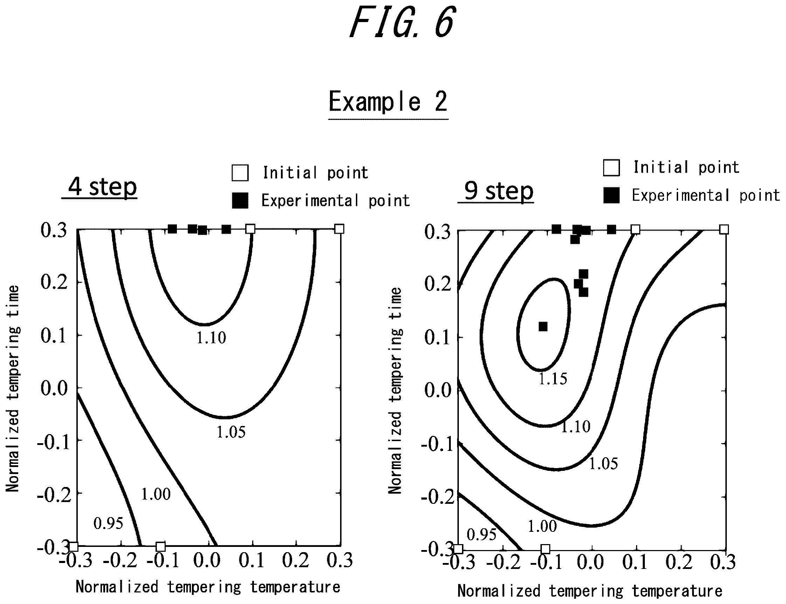

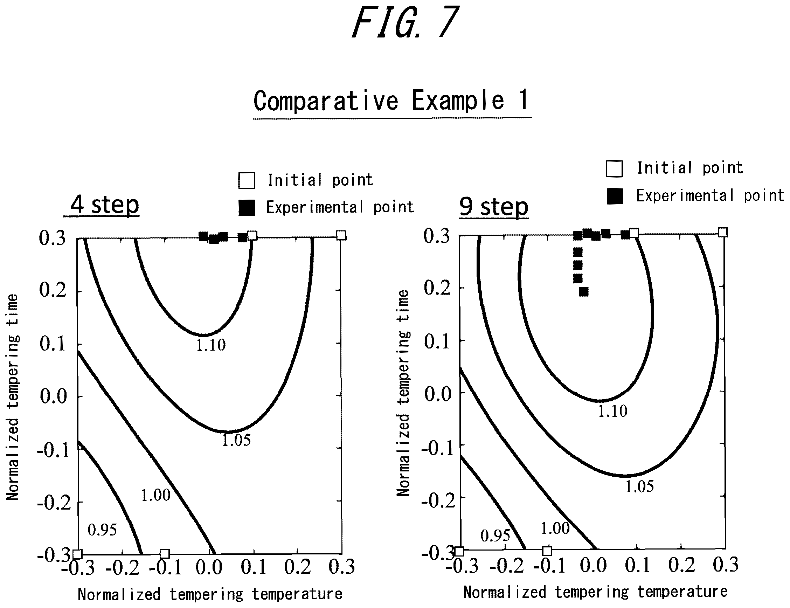

FIG. 5 illustrates the distribution of the predicted property value according to the number of steps for Example 1, FIG. 6 illustrates the same for Example 2, and FIG. 7 illustrates the same for Comparative Example 1. In each drawing, cases where the number of steps is 4 and 9 are illustrated. Further, experimental points are indicated by dots in each drawing.

For Example 1, the property maximum value was found quickly and there was little bias in the selection of the experimental points. Further, for Example 1, the distribution of FIG. 3 provided as the correct answer is largely reproduced.

For Example 2, although slower than Example 1, the property maximum value was found after 9 steps.

In contrast, for Comparative Example 1, it was confirmed that the experimental points were biased and an efficient search was not carried out.

In this way, in this Example Group, it was possible to appropriately select the production condition of the material to have a superior property value to the initial material. Further, it was possible to select a production condition for the material that efficiently maximized the property value with a small number of experimental steps. This enables the efficient selection of production conditions for materials with better property values, and is therefore expected to lead to increased efficiency in materials development, for example. In this Example Group, in the acquisition process (corresponding to step S15), the property values were acquired from a spline interpolation function created based on the property value (El/YS) of 30 points. Here, the property may be maximized using a similar procedure by actually manufacturing samples at selected experimental points and tensile testing the samples to obtain yield stress (YS) and total elongation value (El).

(Example Group 2)

For Example Group 2, the material to be produced was a high tensile steel that is required to have high tensile strength and high ductility (high elongation value). For Example Group 2, the production condition for the high tensile steel capable of achieving both high tensile strength and high elongation value was selected. In the production condition of the high tensile steel, annealing temperature and tempering temperature were changed. There were two combinations of the annealing temperature and the tempering temperature (fifth condition and sixth condition) as follows, and test materials 5 and 6 were produced under the respective conditions.

Under the fifth condition, (normalized annealing temperature, normalized tempering temperature) were set to (-2.36, -1.41). Under the sixth condition, (normalized annealing temperature, normalized tempering temperature) were set to (1.41, 1.41). In order to efficiently find the maximum value of the property, these initial points were set to include the edges of the search space, which is described later.

Here, the normalized annealing temperature was annealing temperature, normalized. The normalization was performed by determining the average value of the annealing temperatures of 25 experimental points in the high tensile steel database obtained so far, subtracting the obtained average value from the annealing temperature, and dividing by the standard deviation. Further, the normalized tempering temperature was tempering temperature, normalized. The normalization was performed by determining the average value of the tempering temperatures of 25 experimental points in the high tensile steel database obtained so far, subtracting the obtained average value from the tempering temperature, and dividing by the standard deviation.

For the test materials 5 and 6, JIS No. 5 test pieces 5 and 6 were produced and subjected to tensile tests to measure the tensile strength (TS) and total elongation value (El) of the test materials 5 and 6. The obtained production conditions of the material and the property values TS and El of the material produced under those production conditions were used as an initial data group (corresponding to step S11). The property values TS and El were also normalized in the same manner as the annealing temperature and tempering temperature. Hereinafter, the property values TS and El are each represented by a normalized value.

Next, a search range was set (corresponding to step S12). In Example Group 2, the ends of the normalized annealing temperature and the normalized tempering temperature of the initial data group were set as the upper and lower limits of the search ranges of the normalized annealing temperature and the normalized tempering temperature. That is, the upper limit of the search range for the normalized annealing temperature was set to 1.41, and the lower limit was set to -2.36. Further, the upper limit of the search range for the normalized tempering temperature was set to 1.41, and the lower limit was set to -1.41.

In order to suppress an increase in the number of experimental steps, the search range was set to be a discrete space divided into intervals of 0.236 for the normalized annealing temperature and intervals of 0.141 for the normalized tempering temperature.

Next, the predicted property value and the prediction error were calculated for TS and El (corresponding to step S13). The predicted property value and the prediction error were calculated using a Gaussian process model using the initial data group.

Next, a calculated value ($A_1$) of the acquisition function of Expression (3) was determined using the initial data group and the obtained predicted property value and prediction error for TS and El. The production condition under which the calculated value ($A_1$) of the acquisition function was maximized was selected as the experimental point (corresponding to step S14). Hereinafter, the selected production condition may be referred to as "production condition (1)".

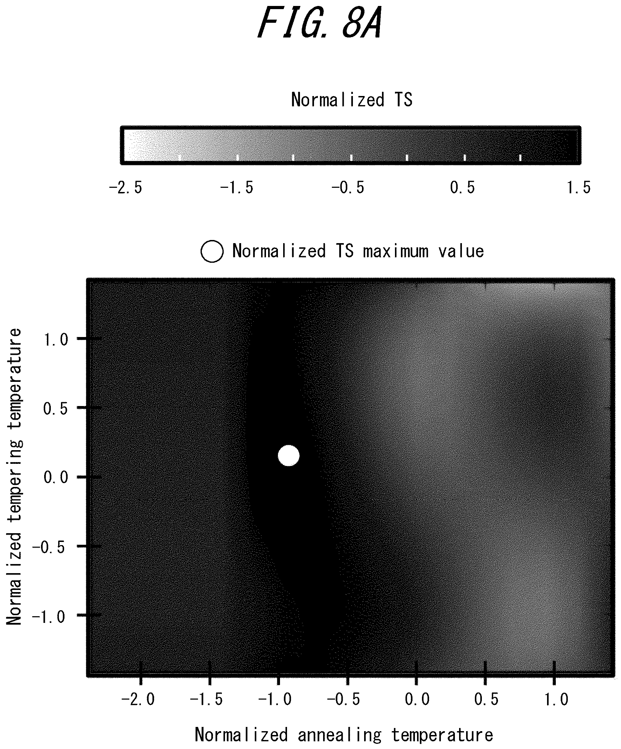

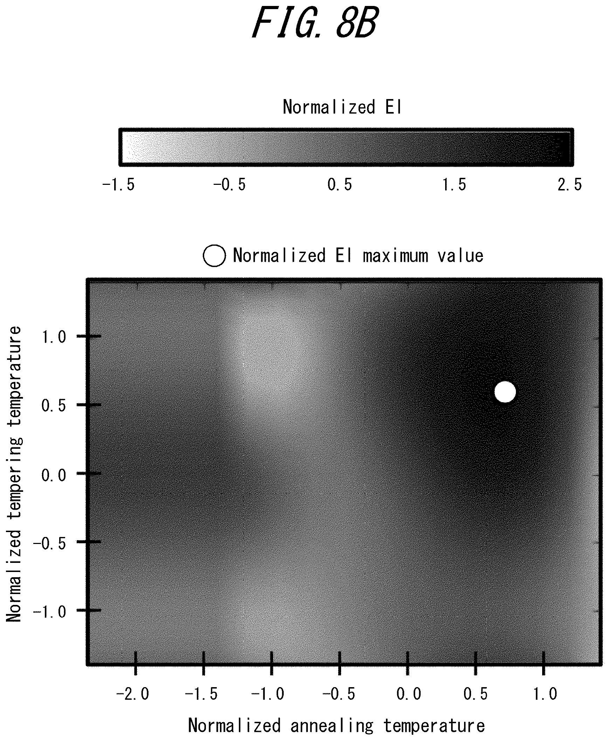

Next, the tensile strength (TS) and the total elongation value (El), which are property values under the production condition of the selected experimental point, were acquired (corresponding to step S15). Hereinafter, the acquired property value may be referred to as "property value (1)". The property value (1) was obtained from FIG. 8A and FIG. 8B illustrated below.

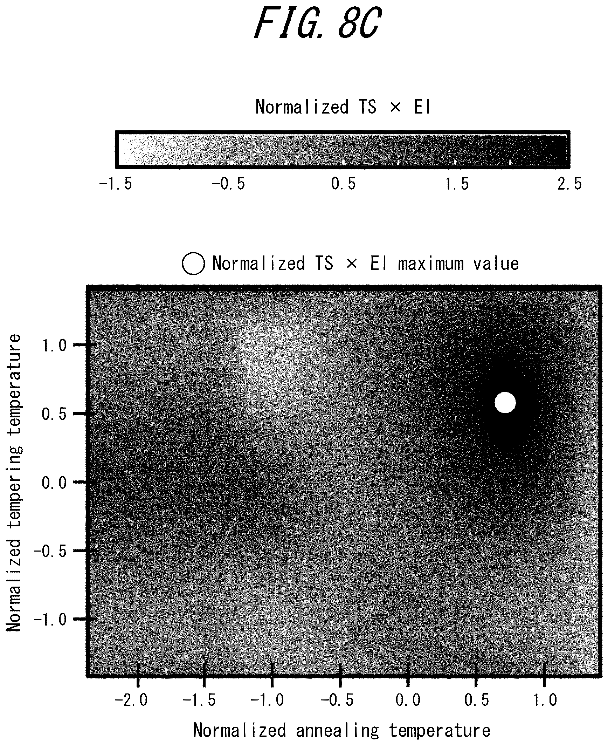

FIG. 8A to FIG. 8C are diagrams illustrating the distribution of the property values based on measured values for Example Group 2, in which FIG. 8A illustrates normalized TS, FIG. 8B illustrates normalized El, and FIG. 8C illustrates normalized TS × El obtained by multiplying TS and El. The normalized TS × El is normalized by multiplying TS and El, subtracting the average value of TS × El obtained, and dividing by the standard deviation.

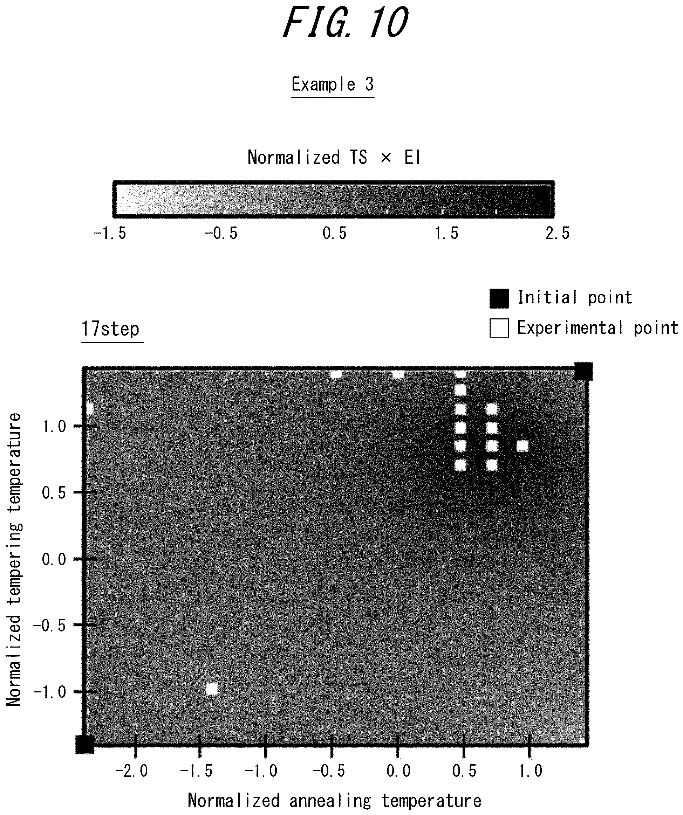

The distributions in FIG. 8A to FIG. 8C were calculated by creating a spline interpolation function based on the property values (TS and El) of 25 points measured experimentally. In FIG. 8A to FIG. 8C and in FIG. 10 to FIG. 13 described later, the distributions of the property values are illustrated in graphs with the horizontal axis representing the normalized annealing temperature and the vertical axis representing the normalized tempering temperature. In FIG. 8A, the maximum value of TS was calculated to be "1.83", and the coordinates (normalized annealing temperature, normalized tempering temperature) corresponding to the property maximum value at this time were (-0.94, 0.14). In FIG. 8B, the maximum value of El was calculated to be "2.30", and the coordinates (normalized annealing temperature, normalized tempering temperature) corresponding to the property maximum value at this time were (0.71, 0.57). Further, in FIG. 8C, the maximum value of TS × El was calculated to be "2.7", and the coordinates (normalized annealing temperature, normalized tempering temperature) corresponding to the property maximum value at this time were (0.71, 0.57). Here, since there was no measurement error, $R_i$ of the acquisition function defined by Expression (4) was set to 1.0.

Next, based on the calculated value $(A_2)$ of the acquisition function obtained after adding the production condition (1) and the property value (1) of the obtained material to the initial data group, and the calculated value $(A_1)$ of the acquisition function described above, it was determined whether or not to search for a production condition to be the next experimental point (corresponding to step S16). Specifically, for the production condition in the search range, the difference between $(A_1)$ and $(A_2)$ was calculated, and the difference was divided by the data points to calculate a normalized difference. Thereafter, when the normalized difference was $1.0 \times 10^{-6}$ or more (equal to or greater than the threshold value), it was determined to be a fail (corresponding to No in step S17), the property value (1) and the production condition (1) were added to the initial data group, and the processing corresponding to the above steps S11 to S16 was executed. Thereafter, the processing corresponding to steps S11 to S16 was repeatedly executed until the normalized difference became less than $1.0 \times 10^{-6}$ (less than the threshold value).

For Example 3, for the acquisition functions of Expressions (3) and (4) used in the selection process, $\varepsilon_{1,i}$ was set to 1.0, and $\varepsilon_{2,i}$ and $\varepsilon_{3,i}$ were set to 2.0. For Example 4, for the acquisition functions of Expressions (3) and (4) used in the selection process, $\varepsilon_{1,i}$ was set to 0.5, and $\varepsilon_{2,i}$ and $\varepsilon_{3,i}$ were set to 2.0.

For Comparative Example 2, UCB (Expressions (5) and (6)) was used as the acquisition function, and the coefficient term $(\varepsilon_{U,i})$ of the prediction error value was set to 0.0. For Comparative Example 3, UCB (Expression (5)) was used as the acquisition function, and the coefficient term $(\varepsilon_{U,i})$ of the prediction error value was set to 6.0. Here, $y_{av,i}$ was the predicted value (predicted property value) of the property value i, and $y_{\sigma,i}$ was the error of the predicted value (predicted property value) of the property value i. Further, max(UCBi) was the maximum value of the acquisition function UCBi corresponding to the property value i.

$$UCB = \prod_{i=1}^{N} \left\{ \frac{UCB_i}{max(UCB_i)} \right\} \quad \ldots \text{Expression (5)}$$

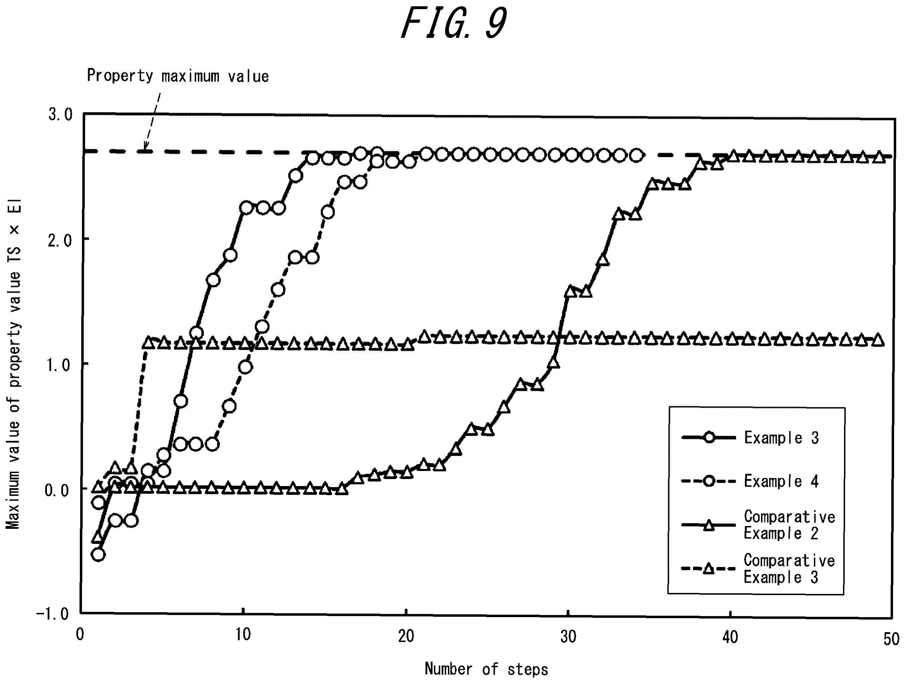

FIG. 9 illustrates the relationship between the number of steps and the maximum value of the property value TS × El when produced under the production condition proposed by this model. The property value TS × El is calculated by multiplying TS and El calculated using the spline interpolation functions illustrated in FIG. 8A and FIG. 8B, respectively, and performance can be evaluated based on whether the property value TS × El reaches a maximum value. The number of steps is the number of times the processing from the input process to the determination process was executed, and may also be called the number of trials. In FIG. 9, for example, when the number of steps is 6, the maximum value of the property values obtained in steps 1 to 6 is indicated. The number of steps required to reach the property maximum value was 40 for Comparative Example 2, and the property maximum value was not found for Comparative Example 3, whereas for Example 3, the property maximum value was approached in 13 steps, and reached in 17 steps. For Example 4, the property maximum value was reached in 21 steps, and it can be seen that the number of steps was fewer for the Examples than the Comparative Examples.

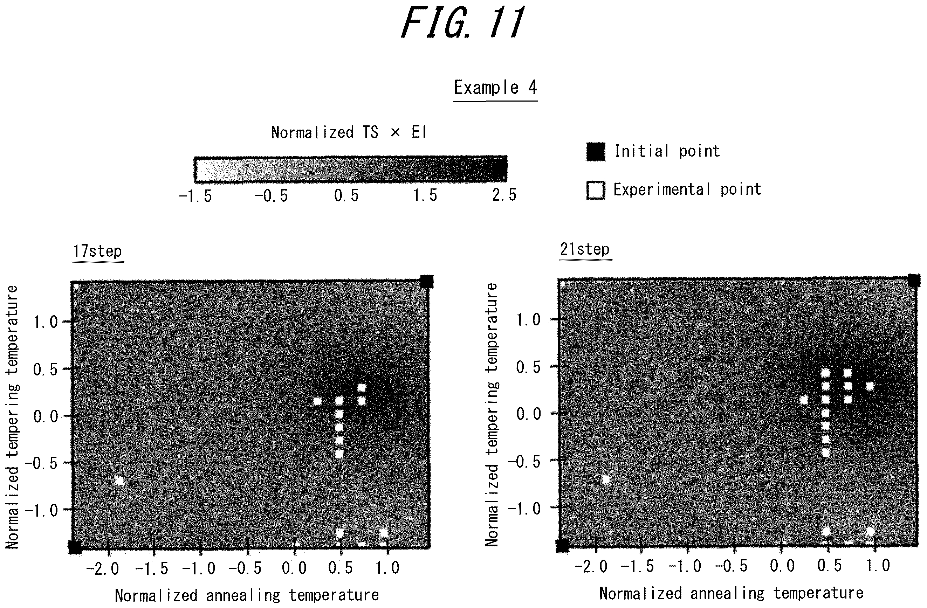

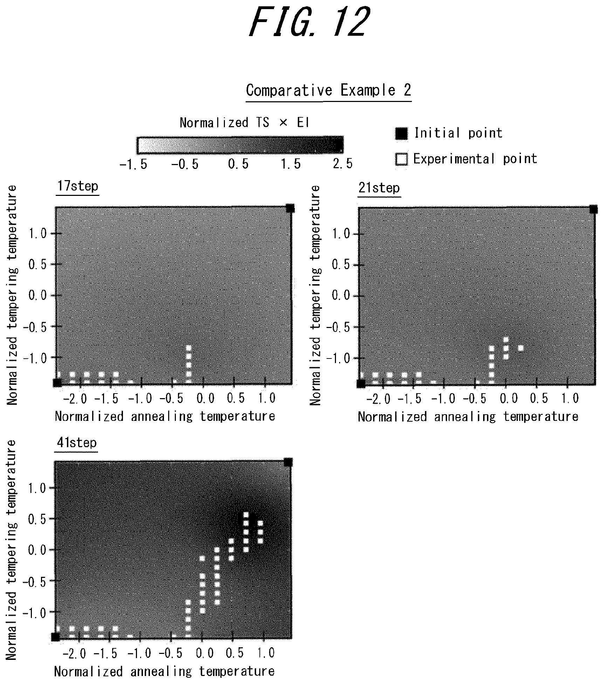

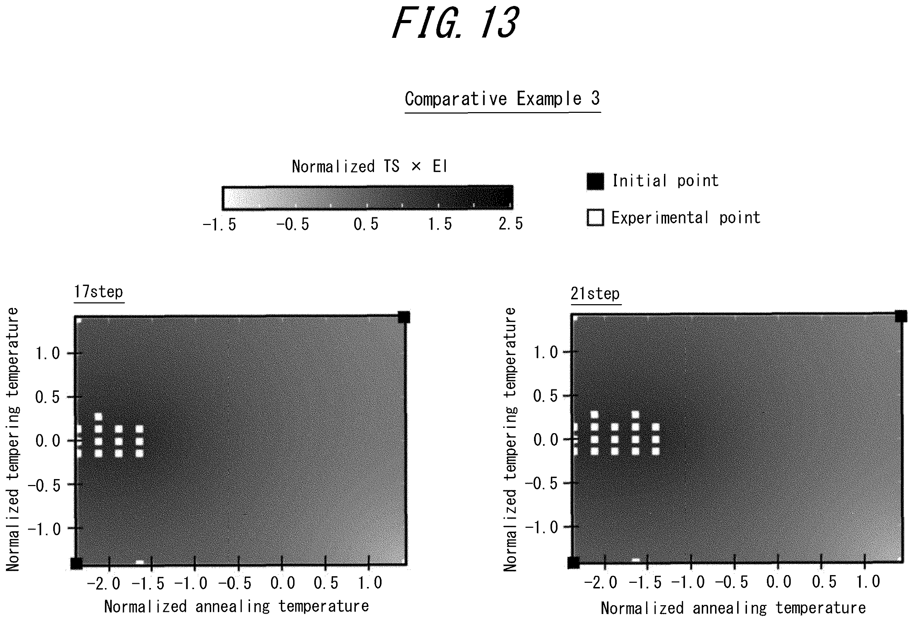

FIG. 10 illustrates the distribution of the predicted property value according to the number of steps for Example 3. FIG. 11 illustrates the distribution of the predicted property value according to the number of steps for Example 4. FIG. 12 illustrates the distribution of the predicted property value according to the number of steps for Comparative Example 2. FIG. 13 illustrates the distribution of the predicted property value according to the number of steps for Comparative Example 3. In each drawing, cases where the number of steps is 17, 21, and 41 are illustrated. For Example 3 in FIG. 10, processing was completed before the 21st step, so the diagrams for the 21st step and the 41st step are not illustrated. For Example 4 in FIG. 11, processing was completed before the 41st step, so the diagram for the 41st step is not illustrated. For Comparative Example 3 in FIG. 13, no improvement in the property value was observed even after 41 steps, so the diagram for 41 steps is not illustrated. Further, experimental points are indicated by dots in each drawing.

For Example 3, the property maximum value was found quickly, and Example 3 was able to largely reproduce the distribution of FIG. 8C, provided as the correct answer.

For Example 4, the property maximum value was found after 21 steps, which was slower than for Example 3.

In contrast, for Comparative Examples 2 and 3, it was confirmed that the experimental points were biased and efficient search was not carried out.

In this way, in Example Group 2, it was possible to appropriately select the production condition of the material to have two property values superior to the property values of the initial material. Further, it was possible to select a production condition for the material that efficiently maximized the property values with a small number of experimental steps. This enables the efficient selection of production conditions for materials with better property values, and is therefore expected to lead to increased efficiency in materials development, for example. In this Example Group, in the acquisition process (corresponding to step S15), the property values were obtained from a spline interpolation function created based on the property values of 25 points. Here, the properties may be maximized using a similar procedure by actually manufacturing samples at selected experimental points and tensile testing the samples to obtain tensile strength (TS) and total elongation value (El).

Although embodiments of the present disclosure have been described based on the drawings and examples, it should be noted that a person skilled in the art may make variations and modifications based on the present disclosure. Therefore, it should be noted that such variations and modifications are included within the scope of the present disclosure. For example, functions and the like included in each component and step may be rearranged, and a plurality of components and steps may be combined into one or divided, as long as no logical inconsistency results. The embodiments according to the present disclosure may be realized as a storage medium on which a program executed by a processor provided to a device is stored. The scope of the present disclosure should be understood to include these examples.

REFERENCE SIGNS LIST

1    production condition selection system
10   production condition selection device
11   input interface
12   output interface
13   processing unit
14   search range setting unit
15   calculation unit
16   selection unit
17   acquisition unit
18   determination unit
30   process computer

**Claims**

1.  A production condition selection method comprising: an input process of inputting an initial data group including initial production condition data and property value data regarding a material to be produced;

    a search range setting process of setting a search range for a production condition of the material based on the initial data group;
    a calculation process of calculating a predicted property value that is a predicted value of a property value of the material in the search range based on the initial data group, and calculating a prediction error that is an estimated error of the predicted property value, using a machine learning model; and
    a selection process of selecting a production condition of the material to be an experimental point based on a calculated value of an acquisition function that uses parameters based on the initial data group, the predicted property value, and the prediction error.

2.  The production condition selection method according to claim 1, further comprising:
    an acquisition process of acquiring a property value of the material under the production condition as the experimental point; and
    a determination process of determining, by comparing calculated values of the acquisition function before and after adding the property value of the material acquired and the production condition as the experimental point to the initial data group, whether or not to select a further production condition of the material to be a new experimental point.

3.  The production condition selection method according to claim 1 or 2, wherein the acquisition function is expressed by the following Expression (1), where A is the calculated value, R is a random number corresponding to a magnitude of a measurement error for correcting the measurement error when measuring the property value of the material, $y_{av}$ is the predicted property value, $y_\sigma$ is an error of the predicted property value, Y is a measured value of the property value, $Y_\sigma$ is a standard deviation of the measured value, max(Y) is the maximum value of the measured value, and $\varepsilon_1$, $\varepsilon_2$, and $\varepsilon_3$ are parameters for correcting the predicted error,

[Math. 1]

$$A = (y_{av} - R \cdot max(Y) - \varepsilon_1 \cdot Y_\sigma) \cdot \int_{R \cdot max(Y)}^{+\infty} \frac{1}{\sqrt{(\varepsilon_2 \cdot y_\sigma)}} exp\left\{-\frac{1}{\varepsilon_3 \cdot y_\sigma}(y - y_{av} - \varepsilon_1 \cdot Y_\sigma)^2\right\} dy + \varepsilon_2 \cdot y_\sigma \cdot \frac{1}{\sqrt{(\varepsilon_2 \cdot y_\sigma)}} exp\left\{-\frac{1}{\varepsilon_3 \cdot y_\sigma}(max(Y) - y_{av} - \varepsilon_1 \cdot Y_\sigma)^2\right\}$$

$$\dots \text{Expression } (1).$$

4. A method of producing a material, the method comprising: producing the material using a production condition selected by the production condition selection method according to any one of claims 1 to 3.

5. A production condition selection device comprising:

an input interface configured to input an initial data group including initial production condition data and property value data regarding a material to be produced;
a search range setting unit configured to set a search range for a production condition of the material based on the initial data group;
a calculation unit configured to calculate a predicted property value that is a predicted value of a property value of the material in the search range based on the initial data group, and calculate a prediction error that is an estimated error of the predicted property value, using a machine learning model; and
a selection unit configured to select a production condition of the material to be an experimental point based on a calculated value of an acquisition function that uses parameters based on the initial data group, the predicted property value, and the prediction error.

6. A program configured to cause a computer to function as:

an input interface configured to input an initial data group including initial production condition data and property value data regarding a material to be produced;
a search range setting unit configured to set a search range for a production condition of the material based on the initial data group;
a calculation unit configured to calculate a predicted property value that is a predicted value of a property value of the material in the search range based on the initial data group, and calculate a prediction error that is an estimated error of the predicted property value, using a machine learning model; and
a selection unit configured to select a production condition of the material to be an experimental point based on a calculated value of an acquisition function that uses parameters based on the initial data group, the predicted property value, and the prediction error.

# FIG. 1

*FIG. 2*

Start

Input process ~S11

Search range setting process ~S12

Calculation process ~S13

Selection process ~S14

Acquisition process ~S15

Determination process ~S16

No — Pass? ~S17

Yes

Output process ~S18

End

# FIG. 3

# FIG. 4

FIG. 5

# FIG. 6

## Example 2

EP 4 682 894 A1

# FIG. 7

## Comparative Example 1

# FIG. 8A

# FIG. 8B

Normalized El

-1.5          -0.5          0.5          1.5          2.5

◯ Normalized El maximum value

Normalized tempering temperature

Normalized annealing temperature

# FIG. 8C

Normalized TS × El

-1.5    -0.5    0.5    1.5    2.5

◯ Normalized TS × El maximum value

# FIG. 9

EP 4 682 894 A1

# FIG. 10

Example 3

Normalized TS × EI

-1.5  -0.5  0.5  1.5  2.5

■ Initial point
□ Experimental point

17step

Normalized tempering temperature

1.0
0.5
0.0
-0.5
-1.0

-2.0  -1.5  -1.0  -0.5  0.0  0.5  1.0

Normalized annealing temperature

FIG. 11

Example 4

FIG. 12

Comparative Example 2

# FIG. 13

Comparative Example 3

EP 4 682 894 A1

## INTERNATIONAL SEARCH REPORT

| | International application No. |
|---|---|
| | **PCT/JP2024/019602** |

| A. | CLASSIFICATION OF SUBJECT MATTER |
|---|---|

*G16C 60/00*(2019.01)i; *G16C 20/70*(2019.01)i
FI: G16C60/00; G16C20/70

According to International Patent Classification (IPC) or to both national classification and IPC

| B. | FIELDS SEARCHED |
|---|---|

Minimum documentation searched (classification system followed by classification symbols)

G16C60/00; G16C20/70

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2023-044459 A (KOBE STEEL, LTD.) 30 March 2023 (2023-03-30) paragraphs [0026]-[0052], [0076]-[0079], [0122]-[0127] | 1, 2, 4-6 |
| A | entire text, all drawings | 3 |
| Y | WO 2023/042612 A1 (NATIONAL UNIVERSITY CORPORATION TOKYO UNIVERSITY OF AGRICULTURE AND TECHNOLOGY) 23 March 2023 (2023-03-23) paragraphs [0008]-[0011], [0034]-[0049], [0052]-[0055] | 1, 2, 4-6 |
| A | entire text, all drawings | 3 |
| Y | 伊藤 聡, マテリアルズ・インフォマティクス開発事例最前線, 第1版, 株式会社エス・ティー・エス 吉田 隆, 31 January 2021, pp. 13, 14, ISBN 978-4-86043-708-4, non-official translation (ITO, Satoshi. Frontline of Materials Informatics Development Case Studies. 1st edition. NTS INC. YOSHIDA: Takashi.) pp. 13, 14 | 1, 2, 4-6 |
| A | pp. 13, 14 | 3 |

✓ Further documents are listed in the continuation of Box C.    ✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **25 July 2024** | **06 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/019602**

## C.    DOCUMENTS CONSIDERED TO BE RELEVANT

| Category* | Citation of document, with indication, where appropriate, of the relevant passages | Relevant to claim No. |
|---|---|---|
| Y | JP 2022-128960 A (FUJITSU LIMITED) 05 September 2022 (2022-09-05) paragraphs [0028]-[0036], [0076]-[0096], [0101]-[0113] | 1, 2, 4-6 |
| A | entire text, all drawings | 3 |
| A | JP 2022-014878 A (JFE STEEL CORPORATION) 20 January 2022 (2022-01-20) entire text, all drawings | 1-6 |
| A | WO 2020/152750 A1 (JFE STEEL CORPORATION) 30 July 2020 (2020-07-30) entire text, all drawings | 1-6 |

Form PCT/ISA/210 (second sheet) (July 2022)

## INTERNATIONAL SEARCH REPORT
### Information on patent family members

International application No.

**PCT/JP2024/019602**

| Patent document cited in search report | | | Publication date (day/month/year) | Patent family member(s) | | | Publication date (day/month/year) |
|---|---|---|---|---|---|---|---|
| JP | 2023-044459 | A | 30 March 2023 | JP | 7201763 | B1 | |
| WO | 2023/042612 | A1 | 23 March 2023 | EP | 4404213 | A1 | |
| | | | | paragraphs [0008]-[0011], [0034]-[0046], [0049]-[0052] | | | |
| JP | 2022-128960 | A | 05 September 2022 | (Family: none) | | | |
| JP | 2022-014878 | A | 20 January 2022 | (Family: none) | | | |
| WO | 2020/152750 | A1 | 30 July 2020 | US | 2022/0100932 | A1 | |
| | | | | EP | 3916651 | A1 | |
| | | | | CN | 113330468 | A | |
| | | | | KR | 10-2021-0114993 | A | |
| | | | | MX | 2021008722 | A | |
| | | | | BR | 112021014096 | A2 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2021101465 A